# EUROPÄISCHE PATENTANMELDUNG

(11) **EP 3 262 930 A1**
(43) Veröffentlichungstag der Anmeldung: **03.01.2018**
(21) Anmeldenummer: 17172182.2
(22) Anmeldetag: 22.05.2017
(51) Int. Cl.: A01K 29/00, A01K 27/00

(54) **SYSTEM ZUR ERFASSUNG DES VITALSTATUS VON TIEREN**

(30) Priorität: 30.06.2016 DE 202016004112 U
(71) Anmelder: Thieme, Andreas, 01896 Pulsnitz (DE); Ursinus, Torsten, 01324 Dresden (DE)
(72) Erfinder: Thieme, Andreas, 01896 Pulsnitz (DE); Ursinus, Torsten, 01324 Dresden (DE)
(74) Vertreter: Kailuweit & Uhlemann Patentanwälte Partnerschaft mbB

(57) **Zusammenfassung**

Die Erfindung betrifft ein System zur Erfassung des Vitalstatus von Tieren, welches zumindest eine erste Vorrichtung zur Erfassung von Vitalfunktionen, und zumindest ein Mittel zur Signalübertragung umfasst, wobei die erste Vorrichtung mindestens zwei voneinander beabstandete erste und/oder zweite Sensoren aufweist. Die Bestandteile des Systems zur Erfassung des Vitalstatus von Tieren sind über Verbindungsstücke miteinander lösbar verbindbar ausgebildet.

Die Erfindung betrifft weiterhin ein Verfahren zur Bestimmung des Vitalstatus von Tieren, wobei die Vitalfunktionen durch eine erste Vorrichtung mit mindestens zwei voneinander beabstandeten ersten und/oder zweiten Sensoren erfasst werden, wobei die erfassten Daten durch zumindest ein Mittel zur Signalübertragung an eine Datenverarbeitungseinrichtung übermittelt, verarbeitet und ausgewertet werden und von der Datenverarbeitungseinrichtung an ein externes Ausgabegerät übermittelt werden, welche den Vitalstatus von Tieren ausgibt.

## Beschreibung

Die Erfindung betrifft ein System zur Erfassung des Vitalstatus von Tieren.

Das System zur Erfassung des Vitalstatus von Tieren umfasst dabei verschiedene Bestandteile wie eine erste Vorrichtung zur Erfassung von Vitalfunktionen und zumindest ein Mittel zur Signalübertragung. Die Bestandteile des Systems zur Erfassung des Vitalstatus von Tieren sind über Verbindungsstücke miteinander lösbar verbindbar ausgebildet.

Sensoren zum GPS Tracking oder zur Messung von Vitalfunktionen finden sich derzeit in Halsbändern, Geschirren oder Kleidungsstücken für Tiere.

Positionsbestimmungen mittels an Geschirren von Tieren angebrachter GPS Technologie werden in etlichen Dokumenten beschrieben. Im Dokument DE 20 2011 109 024 U1 wird bspw. ein am Hundegeschirr fixierbarer Behälter mit GPS Ortungsgerät offenbart In der US 20070204803 A1 wird ein im Halsband eines Tieres angebrachter GPS Sender zur Positionsbestimmung beschrieben.

Im Dokument US 6263836 B1 wird ein Gerät zur Verhaltensüberwachung von Tieren beschrieben, welches diese gleichzeitig durch Reizgebung trainiert und erzieht. Das Gerät wird dabei am Gurtzeug befestigt.

Die EP 2196086 A1 zeigt eine Einrichtung zur Überwachung von Großtieren wie Pferden mit einer Sensoreinrichtung, welche Vitalfunktionen wie Herzfrequenz, Körpertemperatur oder Fellfeuchte misst. Wird beim Überschreiten von Grenzwerten, welche auf einer Speichereinrichtung abgelegt sind, überschritten, wird ein Signal an eine externe Warneinrichtung übermittelt. Die Einrichtung wird an einem Gurtzeug, bestehend aus Bauch-, Brust- und einem zwischen den Beinen verlaufenden Verbindungsgurt, an der Brust des Pferdes gehalten.

Aus der WO 2013/008115 A1 ist ein System mit einem Tierhalsband zur Überwachung der Gesundheit und Vitalzeichen mit Warn- und Diagnosefunktion bekannt. Das Halsband misst Körperfunktionen, wie Atmung, Puls, Temperatur und Bewegung und enthält einen Prozessor, welcher die Ergebnisse interpretiert. Das System kann gemäß einer Ausführungsform der Erfindung auch die eigenen physiologischen Daten mit Vergleichsdaten für die jeweilige Tierrasse vergleichen.

Die DE 10 2014 108 443 A1 offenbart eine Überwachungsvorrichtung für Haus- und Nutztiere, speziell Hunde. Diese umfasst einen Sensor zur Erfassung der Vitalfunktionen (z.B. Herz und Atemfrequenz, Körpertemperatur, Blutzucker) von Tieren wie bspw. Hunden, Katzen oder Pferde und ist an einer Trageeinrichtung (Halsband und/oder Geschirr und/oder Halfter) angebracht. Weiterhin umfasst die Überwachungsvorrichtung eine mit dem Sensor kommunizierende Auswerteeinrichtung. Diese weist auch eine von der Trageeinrichtung getrennte Empfangseinrichtung auf, an welche die gemessenen Daten drahtlos über eine WLAN- oder Bluetooth-Verbindung (bspw. über ein Smartphone) übertragen werden.

In den meisten Schriften ist die Sensor-Ausstattung meist nur an einer Körperstelle des Tieres wie bspw. am Hals durch ein Halsband vorgesehen und nicht am Körper verteilt, wodurch nachteilig keine zuverlässige Messung der Vitalfunktionen garantiert werden kann.

Das Dokument US 20150181840 A1 beschreibt ein Halsband oder Geschirr für Tiere, welches mehrere Sensoren enthält. Die Sensoren messen Parameter zur den Vitalfunktionen und die dabei enthaltenen Daten werden entweder direkt über eine Auswerteeinrichtung im Halsband oder Geschirr ausgewertet oder an einen Datenverwaltungsserver über Ultrabreitbandtechnologie übertragen.

In der US 2008/0255468 A1 ist ein Überwachungs- bzw. Messsystem von physiologischen Parametern bei Säugetieren beschrieben. Dabei sind bspw. Sensoren oder Elektroden zur EKG-Messung in oder an Kleidungsstücken oder Geschirren oder daran befindlichen Taschen oder Rucksäcken an den zu messenden anatomischen Stellen angebracht. Die gemessenen Daten können entweder in Speichermöglichkeiten am Kleidungsstück gespeichert werden oder bspw. drahtlos über Bluetooth-Übertragung zur Echtzeitmessung an Auswerteeinheiten übertragen werden.

In DE 10 2006 027 764 A wird ein Herzschlag-Erfassungs-System für Hunde beschrieben. Dabei wird ein Detektor zum Detektieren der Herzschläge des Tieres an der Position des Herzens durch ein Geschirr gehalten. Der Detektor weist dabei mehrere Elektroden auf, welche am Geschirr angeordnet sind. Das Geschirr, welches einen Brustgurt, Halsband, Nacken- und Sternum-Steg umfasst, hält zudem auch den dazugehörigen Datenspeicher.

Aufgabe ist es, ein System bereitzustellen, welches die Nachteile des Stands der Technik überwindet.

Dabei soll ein System zur Verfügung gestellt werden, welches eine verlässliche Messung der Vitalfunktionen des Tieres in Echtzeit garantiert, indem die Sensorik an anatomisch geeigneten und relevanten Stellen des Tierkörpers angebracht ist. Weiterhin soll das System auf alle Tierarten anwendbar sein.

Zudem soll dem Halter des Tieres eine Kontrolle über den Gesundheitszustand sowie die aktuelle Position des Tieres ermöglicht werden.

Erfindungsgemäß wird die Aufgabe durch ein System gemäß dem unabhängigen Anspruch gelöst. Vorteilhafte Ausgestaltungen der Erfindung sind in den abhängigen Ansprüchen angegeben.

Ein erster Aspekt der Erfindung betrifft System zur Erfassung des Vitalstatus von Tieren, welches erfindungsgemäß zumindest eine erste Vorrichtung zur Erfassung von Vitalfunktionen, und zumindest ein Mittel zur Signalübertragung umfasst, wobei die erste Vorrichtung mindestens zwei voneinander beabstandete erste Sensoren aufweist. Der Vitalstatus umfasst dabei die Vitalfunktionen des Tieres, auch Körperfunktionen oder physiologischen Daten genannt.

In einer Ausführungsform ist das System als System zur Erfassung des Vitalstatus von Tieren ausgebildet. In einer Ausführungsform wird unter dem Begriff Tier jedes Lebewesen verstanden. In einer weiteren Ausführungsform wird unter dem Begriff Tier jedes Wirbeltier verstanden, worunter auch Vögel fallen. Das System zur Erfassung des Vitalstatus ist ausschließlich für Tiere und nicht zur Verwendung am Menschen vorgesehen.

In einer weiteren Ausführungsform wird unter dem Begriff Tier jedes Säugetier verstanden. In einer weiteren Ausführungsform wird unter dem Begriff Tier jedes Tier mit vier Beinen verstanden. Ein Tier mit vier Beinen wird auch als Vierbeiner bezeichnet. Darunter fallen insbesondere auch Gebrauchs- und Nutztiere. Weiterhin umfasst der Begriff Vierbeiner Hunde, Katzen, Pferde, insbesondere Renn- und Sportpferde und Kamele.

Im Folgenden wird das System zur Erfassung des Vitalstatus von Tieren verkürzt als System zur Erfassung des Vitalstatus bezeichnet.

In einer Ausführungsform umfasst das System zur Erfassung des Vitalstatus verschiedene Bestandteile.

Vorteilhaft sind die Bestandteile des Systems modular ausgebildet und können je nach Verwendungszweck oder im Falle von Reparaturen oder Schäden ausgewechselt bzw. ausgetauscht oder nachgerüstet werden. In einer Ausführungsform umfasst das System zur Erfassung des Vitalstatus als Module eine erste Vorrichtung zur Erfassung von Vitalfunktionen, Modul zur Positionsbestimmung, Orientierung und Navigation, ein Modul zur Sprach- und Datenübertragung, Gassensoren, ein Modul zur eindeutigen Besitzeridentifikation sowie ein Modul zur Deaktivierung und Sperrung.

In einer Ausführungsform sind die Bestandteile des Systems zur Erfassung von Vitalfunktionen vorteilhaft gegen Umwelteinflüsse wie beispielsweise Erschütterungen, Vibrationen oder Temperaturextreme geschützt.

In einer Ausführungsform sind die Bestandteile des Systems zur Erfassung von Vitalfunktionen gemäß eines Militärstandards (MIL-STD 810) gegen Staub geschützt.

In einer Ausführungsform umfassen die Bestandteile des Systems zur Erfassung von Vitalfunktionen zumindest eine erste Vorrichtung zur Erfassung von Vitalfunktionen und zumindest ein Mittel zur Signalübertragung.

In einer Ausführungsform weist die erste Vorrichtung zur Erfassung von Vitalfunktionen mehr als zwei voneinander beabstandete erste Sensoren auf, wodurch vorteilhaft mehr Messpunkte und somit eine zuverlässigere Messung der Vitalfunktionen vorgenommen werden kann.

In einer Ausführungsform erfassen die ersten Sensoren die Daten zu den Vitalfunktionen des Tieres. In einer Ausführungsform werden die Daten zu den Vitalfunktionen dabei durch nicht invasive und perkutane Verfahren gemessen, wodurch vorteilhaft das Tier geschont und in seinem Verhalten nicht gestört wird.

Unter den Vitalfunktionen werden lebenswichtige Vorgänge im Wachzustand, der Atmung und des Kreislaufes des Tieres wie beispielsweise die Herzfrequenz, der Blutdruck, die Pulsfrequenz, die Atemfrequenz und die Körpertemperatur verstanden.

In einer bevorzugten Ausführungsform sind die mindestens zwei voneinander beabstandeten ersten Sensoren als Pulsoxysensor und/oder Körpertemperatursensor und/oder Temperatursensor und/oder Schrittzahlsensor und/oder bioelektrischer Widerstandssensor ausgebildet.

In einer Ausführungsform misst der Pulsoxysensor perkutan den Pulsschlag sowie die arterielle Blutstoffsauerstoffsättigung bzw. den Sauerstoffgehalt im Blut des Tieres über die Messung der Lichtabsorption bzw. der Lichtremission. Vorteilhaft sind hierfür die mindestens zwei voneinander beabstandeten Pulsoxysensoren entlang der Herzachsenlagen des Tieres angebracht.

In einer Ausführungsform misst der Körpertemperatursensor die Körpertemperatur des Tieres. Durch das Messen der Körpertemperatur wird vorteilhaft eine Unterkühlung oder Überhitzung des Tieres festgestellt.

In einer Ausführungsform misst der Temperatursensor die Umgebungstemperatur des Tieres. Durch das Messen der Umgebungstemperatur wird vorteilhaft die noch verträgliche Außentemperatur des Tieres festgestellt, wodurch beispielsweise im Falle eines Brandes, frühzeitig eine Warnung übermittelt wird.

In einer Ausführungsform misst der Schrittzahlsensor die Schrittzahlen und somit die zurückgelegte Strecke des Tieres. In einer weiteren Ausführungsform ist der Schrittzahlsensor als Beschleunigungssensor (Accelerometer) ausgebildet.

In einer Ausführungsform misst der bioelektrische Widerstandssensor die Körperzusammensetzung des Tieres. Zu der Körperzusammensetzung zählen Parameter wie Körperwasser, fettfreie Masse, Magermasse, Fettmasse, Körperzellmasse sowie extrazelluläre Masse. In einer Ausführungsform ist der bioelektrische Widerstandssensor als Impedanzsensor ausgebildet.

In einer bevorzugten Ausführungsform weist die erste Vorrichtung weiterhin zumindest zwei voneinander beabstandete zweite Sensoren auf, die von den mindestens zwei voneinander beabstandeten ersten Sensoren verschieden sind. In einer weiteren bevorzugten Ausführungsform sind die ersten Sensoren und die zweiten Sensoren ausgewählt aus einem Pulsoxysensor und/oder Körpertemperatursensor und/oder Temperatursensor und/oder Schrittzahlsensor und/oder bioelektrischer Widerstandssensor. Die Vitalfunktionen des Tieres werden durch die erste Vorrichtung mit mindestens zwei voneinander beabstandeten ersten Sensoren und/oder zweiten Sensoren erfasst. In einer Ausführungsform weisen die zumindest zwei voneinander beabstandeten zweiten Sensoren Messpunkte auf, welche von den zumindest zwei voneinander beabstandeten ersten Sensoren verschieden sind.

In einer Ausführungsform sind die mindestens zwei voneinander beabstandeten ersten und/oder zweiten Sensoren an jeder beliebigen Position des Tieres angebracht. In einer bevorzugten Ausführungsform sind die mindestens zwei voneinander beabstandeten ersten Sensoren entlang der Herzachsenlagen des Tieres angebracht. In einer Ausführungsform beinhaltet die Herzachsenlage die anatomische und/oder elektrische Herzachsenlage des Tieres.

In einer Ausführungsform sind die ersten und/oder zweiten Sensoren in einer Ummantelung fest vergossen. Die Ummantelung entspricht dabei einer Hülse, Hülle, Kapsel oder einem Gehäuse. In einer Ausführungsform besteht die Ummantelung aus Kunststoff oder Glas. Vorteilhaft ist die Ummantelung somit transparent für ein- und austretendes infrarotes Licht. In einer alternativen Ausführungsform sind die ersten und/oder zweiten Sensoren lösbar in einer Ummantelung angeordnet.

In einer Ausführungsform messen und detektieren die ersten und/oder zweiten Sensoren die entsprechenden Daten der Vitalfunktionen drahtlos. In einer Ausführungsform messen die ersten und/oder zweiten Sensoren über eine Infrarot-Schnittstelle, d.h. die ersten und/oder zweiten Sensoren weisen sowohl entsprechende Sender und Empfänger auf. Dabei wird von den ersten und/oder zweiten Sensoren Licht im Infrarot-Bereich ausgesendet, welches an der Haut des Tieres reflektiert und dadurch zurück zu den ersten und/oder zweiten Sensoren gesandt und dort mit den entsprechend gemessenen Informationen empfangen wird. Dabei ist die Geometrie der entsprechenden Ummantelungen der ersten und/oder zweiten Sensoren vorteilhaft derart ausgestaltet, dass sich das vom Sensor generierte infrarote Licht kegelförmig bzw. gerichtet aus dem Sensor ausbreiten kann.

In einer weiteren Ausführungsform ist die Ummantelung dabei wasserdicht und nicht biologisch abbaubar. Vorteilhaft sind die ersten und/oder zweiten Sensoren damit vor Umwelteinflüssen sowie durch die Bewegungen des Tieres, welche eine zusätzliche mechanische Belastung hervorrufen, geschützt. Vorteilhaft ist die Ummantelung transparent ausgestaltet, sodass das von den ersten und/oder zweiten Sensoren ausgesandte Licht durch die Ummantelung hindurch auf die Haut des Tieres trifft und das von dort reflektierte Signal durch die Ummantelung zurück in den Sensor eintreten kann.

In einer Ausführungsform, im Falle eines elektrischen Kabels als Mittel zur Signalübertragung, sind die ersten und/oder zweiten Sensoren lösbar mit dem zumindest einen Mittel zur Signalübertragung verbunden. In einer Ausführungsform ist die lösbare Verbindung als Steckverbindung ausgestaltet. Vorteilhaft können die entsprechenden ersten und/oder zweiten Sensoren vom System zum Austausch und/oder für eine Reinigung und/oder Reparatureinfach entnommen werden.

In einer bevorzugten Ausführungsform umfasst das System zur Messung von Vitalfunktionen zumindest ein Mittel zur Signalübertragung, welches die von den ersten und/oder zweiten Sensoren gemessenen Daten an eine Datenverarbeitungseinrichtung überträgt. In einer weiteren Ausführungsform werden auch die erfassten Daten der anderen Module der zweiten und/oder dritten Vorrichtung durch die Mittel zur Signalübertragung an eine Datenverarbeitungseinrichtung übertragen.

In einer Ausführungsform ist das zumindest eine Mittel zur Signalübertragung als elektrisches Kabel ausgebildet. In einer alternativen Ausführungsform ist das zumindest eine Mittel zur Signalübertragung als eine drahtlose Verbindung wie Bluetooth oder WLAN ausgebildet.

In einer Ausführungsform umfasst das zumindest eine Mittel zur Signalübertragung eine Speichervorrichtung. In einer Ausführungsform ist die Speichervorrichtung beispielsweise als Flash-Speicher oder Speicherkarte, insbesondere als microSD-Speicherkarte, oder als USB-Stick, insbesondere ein microUSB-Stick ausgebildet. Vorteilhaft werden die ermittelten Daten auf der Speichervorrichtung abgelegt und können nachträglich oder zu einem späteren Zeitpunkt ausgewertet werden. Dies ist insbesondere vorteilhaft in Situationen, in denen sich das Tier beispielsweise in Gegenden mit einer schlechten Netzabdeckung aufhält.

In einer weiteren Ausführungsform ist die Speichervorrichtung als prozessgesteuerte Speichereinheit wie beispielsweise als Datenlogger, insbesondere als microSD-Datenlogger ausgebildet. Vorteilhaft wird damit durch die kontinuierliche Datenspeicherung und Ablegung der gemessenen Daten auf einem Speichermedium einem Datenverlust, welcher beispielsweise durch einen Netzausfall oder ein temporär nicht vorhandenes Netz auftreten kann, wodurch die Daten nicht übermittelt werden können, vorgebeugt werden. Die Daten können dann nachträglich ausgewertet werden.

In einer weiteren Ausführungsform umfasst das zumindest eine Mittel zur Signalübertragung ein Audiodatenaufnahme- und wiedergabegerät. In einer Ausführungsform wird durch das Audiodatenaufnahme- und wiedergabegerät die Eingabe bzw. Ausgabe von Sprachkommandos bzw. von akustischen Signalen wiedergegeben. Vorteilhaft kann das Tier dadurch beruhigt werden oder es können Befehle an das Tier weitergeleitet werden, um es zu trainieren oder zu führen. In einer Ausführungsform ist das Audiodatenaufnahme- und wiedergabegerät als MP3-recorder und -player ausgebildet.

In einer weiteren Ausführungsform umfasst das zumindest eine Mittel zur Signalübertragung mindestens ein lichtemittierendes Element In einer weiteren Ausführungsform dient das mindestens eine lichtemittierende Element als Bereitschafts- bzw. Störanzeige der einzelnen ersten und zweiten Sensoren und Module des erfindungsgemäßen Systems. In einer Ausführungsform ist das mindestens eine lichtemittierende Element beispielsweise als Lampe, insbesondere als LED ausgebildet. Durch das mindestens eine lichtemittierenden Element kann sich der Nutzer des Systems den Status des Systems anzeigen lassen. Dies erfolgt beispielsweise durch wechselnde Lichtfarben einer LED.

In einer weiteren Ausführungsform umfasst das zumindest eine Mittel zur Signalübertragung einen Energiespeicher. In einer Ausführungsform ist der Energiespeicher beispielsweise als Ladegerät, insbesondere als Batterie oder Akkumulator ausgebildet. In einer Ausführungsform werden die Datenübermittlungsintervalle in Abhängigkeit der Energieversorgung gesteuert. Vorteilhaft wird der Energiespeicher überwacht und eine situationsbedingte Abschaltung bestimmter Module erzwungen, um beispielsweise die Laufzeit des Energiespeichers zu erhöhen.

In einer Ausführungsform ist der Energiespeicher mit einer Möglichkeit zur Energieerzeugung verbunden. In einer Ausführungsform ist die Möglichkeit zur Energieerzeugung als Solarmodul ausgebildet, wodurch vorteilhaft eine autarke Versorgung des erfindungsgemäßen Systems gewährleistet ist.

In einer bevorzugten Ausführungsform umfasst das System zur Erfassung des Vitalstatus weiterhin eine zweite Vorrichtung zur Positions- und Standortbestimmung von Tieren. Vorteilhaft ist durch die zweite Vorrichtung eine exakte Ortung und Geodatenübermittlung des Tieres gewährleistet ist. Somit ist der Benutzer stets über den aktuellen Aufenthaltsort des Tieres informiert.

In einer Ausführungsform umfasst die zweite Vorrichtung zur Positions- und Standortbestimmung ein Modul zur Positionsbestimmung, Orientierung und Navigation. In einer Ausführungsform ist das Modul zur Positionsbestimmung, Orientierung und Navigation beispielsweise als GPS, GNSS oder als Gyroskop ausgebildet. In einer alternativen Ausführungsform ist das Positionsbestimmung, Orientierung und Navigation, bei fehlender GPS-Verbindung, als GSM-Ortung durch das Mobilfunknetz, wie beispielsweise als Cell-Tower-Triangulation, durch ein GSM-Modul ausgebildet. Das GSM-Modul kann beispielsweise als SIM-Karte ausgeführt sein.

In einer weiteren Ausführungsform wird durch das im Modul zur Positionsbestimmung, Orientierung und Navigation ausgebildete GNSS eine Geschwindigkeitsbestimmung des Tieres gewährleistet.

In einer weiteren Ausführungsform ist durch das im Modul zur Positionsbestimmung, Orientierung und Navigation ausgebildete GPS und GNSS weiterhin eine Höhenmessung möglich (GPS-Levelling), wodurch vorteilhaft der Standort des Tieres in Abhängigkeit von seiner Höhenlage wiedergegeben wird.

In einer Ausführungsform umfasst die zweite Vorrichtung zur Positions- und Standortbestimmung ein Modul zur Sprach- und Datenübertragung. In einer Ausführungsform ist das Modul zur Sprach- und Datenübertragung, beispielsweise als GSM/3G-Verbindung ausgebildet. In einer alternativen Ausführungsform ist das Modul zur Sprach- und Datenübertragung, bei fehlender GSM/3G-Verbindung, als Telekommunikationsdienst zur Übertragung von Kurznachrichten im Mobilfunknetz wie beispielsweise SMS, ausgebildet.

In einer Ausführungsform umfasst die zweite Vorrichtung zur Positions- und Standortbestimmung von Tieren einen Kompass. Vorteilhaft gibt der Kompass Aufschluss über die Richtung, in welche sich das Tier bewegt.

In einer Ausführungsform umfasst die zweite Vorrichtung zur Positions- und Standortbestimmung von Tieren ein optoelektronische Detektionsmittel. Vorteilhaft wird durch das optoelektronische Detektionsmittel eine visuelle Kontrolle der Umgebung des Tieres und somit eine Positionsbestimmung ermöglicht. In einer Ausführungsform ist das optoelektronische Detektionsmittel als Kamera ausgebildet. In einer weiteren Ausführungsform ist das optoelektronische Detektionsmittel als Videokamera ausgebildet. Vorteilhaft erhält der Halter des Tieres einen Eindruck von der Umgebung des Tieres in Echtzeit ohne Sichtkontakt zum Tier. Zu der Umgebung zählen sowohl Gefahrenbereiche als auch Landschaftsaufnahmen.

In einer Ausführungsform umfasst die zweite Vorrichtung zur Positions- und Standortbestimmung von Tieren mindestens ein Leuchtmittel. Das Leuchtmittel dient vorteilhaft der besseren Ausleuchtung der Umgebung des Tieres, wodurch eine bessere Orientierung des Tieres, speziell in dunkler Umgebung, möglich ist. In einer Ausführungsform ist das mindestens eine Leuchtmittel beispielsweise als Lampe, insbesondere als LED ausgebildet.

In einer Ausführungsform wird durch das von der zweiten Vorrichtung zur Positions- und Standortbestimmung von Tieren umfassende Modul zur Positionsbestimmung, Orientierung und Navigation ausgebildete GPS eine dem Tier selbstständig folgende Drohne angesteuert (beispielsweise eine AirDog-Drohne, https://www.airdog.com/), wodurch der Halter den genauen Aufenthaltsort des Tieres auch visuell beobachten kann. Vorteilhaft können dadurch Tiere, welche im Rettungseinsatz ausgebildet werden wie beispielsweise Rettungshunde, kontrolliert in Gebiete geschickt werden, welche aufgrund fehlenden Blickkontaktes zum Halter des Tieres und somit einer nicht gewährbaren Sicherheit des Tieres im Normalfall nicht mehr zugänglich sind.

In einer bevorzugten Ausführungsform umfasst das System eine dritte Vorrichtung, ausgewählt aus Gassensoren und/oder einem Modul zur eindeutigen Besitzeridentifikation und/oder einem Modul zur Deaktivierung und Sperrung.

In einer Ausführungsform umfasst die dritte Vorrichtung des Systems Gassensoren. In Verbindung mit der Drohne ist auch hier ein Schicken des für den Rettungseinsatz ausgebildeten Tieres unter kontrollierter Beobachtung in Gefahrengebiete wie beispielsweise Brandherde möglich, wodurch die Zusammensetzung der Luft hinsichtlich giftiger Gase zu messen. In einer Ausführungsform sind die Gassensoren als CO₂-Sensoren ausgebildet.

In einer weiteren Ausführungsform umfasst die dritte Vorrichtung des Systems ein Modul zur eindeutigen Besitzeridentifikation mittels ICCID, IMSI oder Seriennummern.

In einer weiteren Ausführungsform umfasst die dritte Vorrichtung des Systems ein Modul zur Deaktivierung und Sperrung der anderen Module. In einer Ausführungsform geschieht dies über eine Kommandofunktion, eine App oder einen Alarm. Vorteilhaft wird somit Diebstahl oder einer Fremdnutzung entgegengewirkt.

In einer bevorzugten Ausführungsform werden die Daten zu den Vitalfunktionen des Tieres durch die zweite Vorrichtung und/oder die dritte Vorrichtung erfasst.

In einer bevorzugten Ausführungsform umfasst das System zur Erfassung des Vitalstatus weiterhin eine Positionierungseinrichtung.

In einer bevorzugten Ausführungsform ist die Positionierungseinrichtung als Stabilisierungsvorrichtung zur medizinischen Behandlung, zur Entlastung, Stabilisation, Sicherung sowie dem waagerechten Anheben und Transport von Tieren ausgestaltet. Bevorzugt umfasst die Positionierungseinrichtung mindestens ein Bauchelement und ein Rückenelement, welche durch Verbindungselemente lösbar verbindbar ausgestaltet sind.

In einer Ausführungsform weist die Positionierungseinrichtung zumindest teilweise reflektierende Elemente auf. Vorteilhaft sind die reflektierenden Elemente an der in die Umgebung zeigende Oberfläche der Positionierungseinrichtung angebracht, sodass der Träger der Positionierungseinrichtung durch die Reflexion im Straßenverkehr auch im Dunkeln oder bei schlechten Lichtverhältnissen gut erkennbar ist.

In einer Ausführungsform ist mindestens ein Bestandteil des Systems zur Erfassung von Vitalfunktionen an der Positionierungsvorrichtung angeordnet. In einer bevorzugten Ausführungsform sind die mindestens zwei voneinander beabstandeten ersten und/oder zweiten Sensoren und/oder das zumindest eine Mittel zur Signalübertragung an der Positionierungsvorrichtung angeordnet.

In einer bevorzugten Ausführungsform ist die erste Vorrichtung mit den mindestens zwei voneinander beabstandeten ersten und/oder zweiten Sensoren punktuell oder flächendeckend in der Positionierungseinrichtung eingebracht sind. In einer weiteren Ausführungsform sind neben der ersten Vorrichtung mit den mindestens zwei voneinander beabstandeten ersten und/oder zweiten Sensoren weiterhin das Mittel zur Signalübertragung und/oder die zweite Vorrichtung zur Positions- und Standortbestimmung und/oder die dritte Vorrichtung in der Positionierungseinrichtung eingebracht.

In einer Ausführungsform weist die Positionierungseinrichtung Aussparungen für die mindestens zwei voneinander beabstandeten ersten und/oder zweiten Sensoren auf. Vorteilhaft können die Sensoren durch diese Aussparungen geschoben werden und arbeiten sich durch die Bewegung des Tieres bis an die Oberfläche dessen Haut ein, um durch diesen hergestellten Kontakt zur Haut des Tieres zuverlässig Daten aufnehmen zu können. Dabei arbeitet sich der erste und/oder zweite Sensor durch Felle und Federn verschiedener Länge bis zur Oberfläche der Haut des jeweiligen Tier durch, ohne Druckstellen zu hinterlassen. Je nach Beschaffenheit der äußeren Oberfläche des Tieres ist es auch möglich, verschiedene Formen der Ummantelungen der ersten und/oder zweiten Sensoren zu wählen. So können die Ummantelungen beispielsweise eine abgeflachte, runde oder konische Form aufweisen. Die Oberfläche der Ummantelung ist glatt ausgestaltet, um keine Druckstellen oder Verletzungen beim Tier zu verursachen. Zur Befestigung und Arretierung weist die entsprechende Ummantelung der Sensoren dabei beispielsweise eine erste Scheibe auf, welche bei Durchführung des Sensors durch die Aussparung der Positionierungseinrichtung in Kontakt mit der nach außen weisenden Seite der Positionierungseinrichtung ist und mit einer zweiten Scheibe zum Gegenklemmen, welche sich an der Innenseite, d.h. zur Tier zeigenden Seite, der Positionierungseinrichtung befindet, fixiert wird.

Vorteilhaft befinden sich so die an der Positionierungseinrichtung eingebrachten mindestens zwei voneinander beabstandeten ersten und/oder zweiten Sensoren in Kontakt mit der Haut des Tieres.

In einer alternativen Ausführungsform sind die mindestens zwei voneinander beabstandeten ersten und/oder zweiten Sensoren in die Positionierungseinrichtung durch Weben, Stricken, Flechten, Kleben oder Drucken eingebracht. Vorteilhaft sind die ersten und/oder zweiten Sensoren so durch Witterungseinflüsse geschützt.

In einer Ausführungsform ist das optoelektronische Detektionsmittel an der Positionierungseinrichtung angebracht. Vorteilhaft wird das dabei erzeugte Bild auf Höhe des das optoelektronische Detektionsmittel tragenden Tieres aufgenommen, wodurch man denselben Blickwinkel wie das Tier hat. Dies ist insbesondere im Fall von Rettungstieren, wie Rettungshunden, relevant, da diese auch in unwegsamen Gelände oder Höhlen vordringen müssen, und so der Halter über etwaige Besonderheiten besser informiert wird, als wenn das optoelektronische Detektionsmittel als Aufsatz auf der Positionierungseinrichtung angebracht ist.

In einer Ausführungsform ist die Positionierungseinrichtung zumindest teilweise flächig ausgebildet und weist mindestens eine flexible und anatomisch anpassbare Auflagefläche auf. Dabei heißt anatomisch anpassbar, dass sich die Positionierungseinrichtung an die Körperbereiche des Tieres flexibel anschmiegt. Vorteilhaft wird dadurch der Tragekomfort gesteigert. Weiterhin vorteilhaft wird durch die Ausgestaltung der Positionierungseinrichtung beispielweise eine muskel-, gelenk- und blutgefäßschonende Behandlung des Tieres gewährleistet. Somit wird der Bewegungsapparat des Tieres geschont.

In einer Ausführungsform verläuft die Positionierungseinrichtung entlang anatomisch geeigneter Positionen des Tieres. Unter anatomisch geeigneten Positionen sind jene Positionen zu verstehen, welche das Tier in seiner Bewegung nicht hindern oder schmerzen, wodurch aber die Vitalfunktionen vorteilhaft direkt und in Echtzeit gemessen werden können. Durch das Anliegen der Positionierungseinrichtung an anatomisch geeigneten Positionen des Tieres werden weiterhin vorteilhaft die unterschiedlich ausgebildeten Fettstrukturen und Fettsättigungen in der Haut des Tieres berücksichtigt, sodass die Vitalwertfunktionen exakt ermittelt werden können. In einer Ausführungsform verläuft die Positionierungseinrichtung entlang anatomisch geeigneter Positionen des Rumpfes des Tieres. In einer Ausführungsform verläuft die Positionierungseinrichtung mit den daran eingebrachten ersten und/oder zweiten Sensoren über anatomisch relevante Körperstellen des Tieres wie beispielsweise entlang der Herzachsenlagen, wodurch vorteilhaft eine verlässliche Messung der Vitalfunktionen erfolgt

In einer Ausführungsform weist die Positionierungseinrichtung ein robustes textiles Material auf. In einer Ausführungsform weist das robuste textile Material eine Wassersäule von 3000 bis 4000 mmH₂O, bevorzugt 3500 mmH₂O auf. In einer weiteren Ausführungsform weist das robuste textile Material der Stabilisierungsvorrichtung Zugfestigkeiten im Bereich von 1 bis 5 kg/cm auf. Vorteilhaft wird die Zugfestigkeit des robusten textilen Materials in Abhängigkeit von der Größe des Hundes und somit von der Größe den jeweiligen Bauch-, Brust-, Schulter- und Rückenelementen gewählt. Das robuste textile Material der Positionierungseinrichtung ist dabei so ausgestaltet, dass die Positionierungseinrichtung schmutzabweisend, waschbar und atmungsaktiv ist.

In einer Ausführungsform ist die Positionierungseinrichtung zumindest teilweise gepolstert. In einer weiteren Ausführungsform weist die Positionierungseinrichtung zumindest teilweise eine Temperiervorrichtung auf. In einer Ausführungsform ist die Temperiervorrichtung beheizbar und/oder kühlbar ausgebildet.

In einer bevorzugten Ausführungsform ist die Positionierungseinrichtung als handelsübliches Geschirr ausgebildet. In einer weiteren Ausführungsform ist die Positionierungseinrichtung als handelsübliches Gurtsystem ausgebildet. In einer weiteren Ausführungsform ist die Positionierungseinrichtung als handelsübliches Gurtband ausgebildet.

In einer Ausführungsform ist das zumindest eine Mittel zur Signalübertragung, wenn es als elektrisches Kabel ausgebildet ist, in der Positionierungseinrichtung fest verbaut. Dabei verläuft das Mittel zur Signalübertragung insbesondere entlang der Kante die Positionierungseinrichtung. In einer Ausführungsform ist die Kante der Positionierungseinrichtung aus einem stabilen Stoff gefertigt. In einer Ausführungsform ist die Kante der Positionierungseinrichtung aus Leder, wie Echt- oder Kunstleder, oder Neopren gefertigt. Das zumindest eine Mittel zur Signalübertragung verläuft damit von außen geschützt in der Positionierungseinrichtung. Vorteilhaft ist die Positionierungseinrichtung dadurch waschbar, ohne das Mittel zur Signalübertragung vorher entfernen zu müssen.

In einer Ausführungsfirm ist die Positionierungseinrichtung individuell in variablen und unterschiedlichen Abmessungen, je nach Größe des Tieres, gestaltbar und miteinander kombinierbar. Vorteilhaft wird dabei auf die Variabilität in der Körperbeschaffenheit wie Größe und das Gewicht aufgrund der Vielzahl Tierrassen geachtet. Die unterschiedliche Physis der Tiere führt zu entsprechend anderen Vitalfunktionen, wie stark variierenden Puls- und Atemfrequenzen.

In einer bevorzugten Ausführungsform sind die Bestandteile des Systems zur Erfassung von Vitalfunktionen mittels Verbindungsstücke mit der Positioniereinrichtung lösbar verbindbar ausgebildet. In einer weiteren Ausführungsform sind dabei insbesondere die ersten und/oder zweiten Sensoren mittels Verbindungsstücke mit der Positioniereinrichtung lösbar verbindbar ausgebildet. In einer Ausführungsform umfassen die Verbindungsstücke mechanische Verbindungsstücke wie Schnallen, insbesondere Rollschnallen, Doppelsteg-Schnallen, Dreisteg-Schnallen, Leiterschnallen, Schiebeschnallen, Klemmschnallen, Klickschnallen (Blitzverschluss, auch Klickschließen genannt), Steckschnallen oder Formschlussschnallen. In einer weiteren Ausführungsform umfassen die Verbindungsstücke mechanische Verbindungsstücke wie Knebelverschlüsse, Klettverschlüsse, Druckknöpfe oder Reißverschlüsse. Vorteilhaft sind die Bestandteile des Systems zur Erfassung von Vitalfunktionen somit stabil an der Positionierungseinrichtung angebracht und können von dieser leicht entfernt bzw. ersetzt werden.

In einer bevorzugten Ausführungsform werden die von den ersten und/oder zweiten Sensoren der ersten Vorrichtung gemessenen Daten der Vitalfunktionen des Tieres über das Mittel zur Signalübertragung in eine Datenverarbeitungseinrichtung übermittelt, dort verarbeitet und ausgewertet. Beispielsweise wird eine Leistungskurve der Aktivitäten des Tieres erstellt. In einer weiteren Ausführungsform werden die von der zweiten Vorrichtung und/oder dritten Vorrichtung gemessenen Daten des Tieres über das Mittel zur Signalübertragung in eine Datenverarbeitungseinrichtung übermittelt, dort verarbeitet und ausgewertet.

In einer Ausführungsform umfasst die Datenverarbeitungseinrichtung einen Prozessor, welcher über verschiedene Signalleitungen mit anderen Elementen der Datenverarbeitungseinrichtung wie beispielsweise einem Arbeitsspeicher oder einem GSM-Modul zur Übermittlung von Daten in eine Cloud verbunden ist.

In einer alternativen Ausführungsform ist die Datenverarbeitungseinrichtung als Cloud ausgebildet. Vorteilhaft werden die von den ersten und/oder zweiten Sensoren gemessenen Daten durch Mittel zur Signalübertragung, welche drahtlos ausgestaltet sind, in die Cloud gesendet werden. Vorteilhaft kann somit der Zustand der Tieres sowie seine Position in Echtzeit vom Nutzer abgerufen werden.

In einer Ausführungsform umfasst das System zur Bestimmung des Vitalstatus von Tieren weiterhin ein Gehäuse zur Aufnahme von Bestandteilen des Systems sowie zum Schutz vor mechanischen Einflüssen. In dem Gehäuse ist zumindest ein Teil der Bestandteile Systems angebracht. In einer Ausführungsform sind in dem Gehäuse zumindest Teile der Mittel zur Signalübertragung und/oder zumindest Teile der ersten Vorrichtung zur Erfassung von Vitalfunktionen und/oder zumindest Teile der zweiten Vorrichtung zur Positions- und Standortbestimmung und/oder zumindest Teile der dritten Vorrichtung angebracht. In einer Ausführungsform ist in dem Gehäuse der Energiespeicher und/oder die Speichervorrichtung und/oder lichtemittierende Elemente und/oder das GSM-Modul und/oder der Kompass und/oder der Arbeitsspeicher angebracht.

In einer Ausführungsform ist das Gehäuse an der äußeren Seite der Positionierungsvorrichtung angeordnet. Dabei erfolgt die Anordnung durch schnell lösbare Verbindungen wie beispielsweise Schnappverbindungen, Klettverschlüsse oder auch Schraubverbindungen. Vorteilhaft ist es dadurch vom Benutzer einfach und schnell von der Positionierungsvorrichtung zu entnehmen.
In einer Ausführungsform ist das Gehäuse starr und/oder wasserdicht und/oder stabil ausgebildet. Vorteilhaft werden die darin enthaltenen Bestandteile vor Umwelteinflüssen und Erschütterungen geschützt. In einer weiteren Ausführungsform ist das Gehäuse nicht biologisch abbaubar ausgebildet. In einer weiteren Ausführungsform werden äußerlich auftretende Schwingungen durch die Ausgestaltung des Gehäuses abgeschwächt und somit eine Dämpfung des Gehäuses erreicht.

Dabei sind die einzelnen Gehäuseteile durch Dichtungen miteinander verbunden. Bevorzugt ist das Gehäuse zweiteilig ausgeführt. Vorteilhaft ist das Gehäuse leicht zu öffnen, sodass die entsprechenden darin enthaltenen Bestandteile im Falle von Defekten oder für eine Reparatur ausgetauscht und zum Nachrüsten eingebracht werden können. In einer Ausführungsform besteht das Gehäuse aus mehr als einem Teil.

In einer Ausführungsform besteht das Gehäuse aus einem Epoxidharz. Vorteilhaft ist das Gehäuse aus einem transparenten Epoxidharz gebildet, sodass Einblicke auf die darin enthaltenen Bestandteile des Systems gewährt werden. Insbesondere bei im Gehäuse angeordneten lichtemittierenden Elementen kann sich der Nutzer des Systems den Status des Systems anzeigen lassen.

In einer bevorzugten Ausführungsform werden die von der ersten Vorrichtung gemessenen Daten der Vitalfunktionen des Tieres von der Datenverarbeitungseinrichtung an ein externes Ausgabegerät übermittelt. In einer bevorzugten Ausführungsform gibt das externe Ausgabegerät den Vitalstatus von Tieren aus. In einer weiteren Ausführungsform gibt das externe Ausgabegerät die Daten zu Position und Standort der zweiten Vorrichtung aus. In einer weiteren Ausführungsform gibt das externe Ausgabegerät die von der dritten Vorrichtung ermittelten Daten aus.

In einer weiteren Ausführungsform werden die Daten der Vitalfunktionen über eine drahtlose Verbindung wie Bluetooth oder WLAN von der Datenverarbeitungseinrichtung an das externe Ausgabegerät übertragen.

In einer Ausführungsform ist das externe Ausgabegerät als Computer, Tablet, Laptop oder Smartphone gestaltet. Vorteilhaft erfolgt eine benutzerfreundliche Bedienung des externen Ausgabegerätes durch speziell programmierte Apps. In einer weiteren Ausführungsform werden die Daten der Vitalfunktionen durch das externe Ausgabegerät visualisiert und als Übersichten und Statistiken dargestellt.

In einer Ausführungsform weist das externe Ausgabegerät ein Computerprogrammprodukt auf, welches die Daten der Vitalfunktionen auswertet. Vorteilhaft werden dabei die Aktivität, der Allgemeinzustand sowie der Gesundheitszustand des Tieres berücksichtigt.

In einer Ausführungsform gibt das externe Ausgabegerät ein Warnsignal bei Über- oder Unterschreitung von Vitalfunktions-Normwerten ab. Vorteilhaft erfolgt somit eine Überwachung des Tieres in Echtzeit, wodurch rechtzeitig ein Eingriff beispielsweise in die Bewegung oder Nahrungsaufnahme des Tieres vorgenommen werden kann.

In einer bevorzugten Ausführungsform ist das externe Ausgabegerät so gestaltet, dass es Befehle an die Datenverarbeitungseinrichtung zurücksendet, welche wiederum an die ersten und/oder zweiten Sensoren übermittelt werden.

In einer Ausführungsform werden die erfassten Daten von der zweiten Vorrichtung und/oder dritten Vorrichtung durch zumindest ein Mittel zur Signalübertragung an eine Datenverarbeitungseinrichtung übermittelt, verarbeitet und ausgewertet und von der Datenverarbeitungseinrichtung an ein externes Ausgabegerät übermittelt, welche den Vitalstatus von Tieren ausgibt.

Durch die modulare Zusammensetzung des Systems ist es vorteilhaft je nach Bedarf und Situation möglich, einzelne Module für die Datenerfassung hinzuzuschalten oder auszuschalten. Dadurch kann Energie gespart werden und ungewollte, überflüssig angesammelte Daten vermieden werden. Will der Halter des Tieres das System beispielsweise nur zur Standortbestimmung des Tieres verwenden, benötigt er beispielsweise nicht die erste Vorrichtung zur Erfassung von Vitalfunktionen.

Die durch die mindestens zwei voneinander beabstandeten ersten und/oder zweiten Sensoren erfassten Daten der Vitalfunktionen sind beispielsweise für den Halter des Tieres insofern interessant, als dass er gewisse Stressfaktoren im Umgang mit dem Tier berücksichtigen kann. So kann zum Beispiel der Halter eines Hundes beim Radfahren in Echtzeit die Belastung des neben des Fahrrades laufenden Hundes beobachten und einschätzen, wann die Belastungsgrenze des Hundes erreicht ist.

Weiterhin sind die durch die mindestens zwei voneinander beabstandeten ersten und/oder zweiten Sensoren erfassten Daten der Vitalfunktionen auch für Veterinärmediziner, Pharmahersteller sowie für tiermedizinische Studien und Untersuchungen interessant. Vorteilhaft kann durch das erfindungsgemäße System der Energiehaushalt des Tieres untersucht werden. Indem der Veterinärmediziner weitere Informationen zum Verhalten des Tieres wie beispielsweise wann und was das Tier frisst und/oder trinkt, kann in Verbindung mit den gemessenen Daten der Vitalfunktionen eine Einschätzung zum weiteren Umgang mit dem Tier gegeben werden. Weitere interessante Ist-Größen, wie Übersichten zu den Bewegungszeiten des Tieres oder Medikamenteneinnahmen können ebenfalls zur Beurteilung und besseren Behandlung oder Therapie des Tieres herangezogen werden.

In einer Ausführungsform wird durch das erfindungsgemäße System zur Erfassung des Vitalstatus eine Überwachung des Wohlbefindens des Tieres gewährleistet. Vorteilhaft ist der Halter des Tieres über den Gesundheitszustand somit das Befinden des Tieres in Echtzeit informiert. Auftretende Stressfaktoren, welche sich in den Daten der Vitalfunktionen wiederspiegeln wie beispielsweise eine erhöhte Herz- oder Atemfrequenz und somit Über- oder Unterschreitung der Normwerte, können dabei analysiert und ausgewertet und somit eine individuelle Behandlung des Tieres vorgenommen werden.

Für die Realisierung der Erfindung ist es auch zweckmäßig, die vorbeschriebenen erfindungsgemäßen Ausgestaltungen, Ausführungsformen und Merkmale der Ansprüche in jeder Anordnung miteinander zu kombinieren.

### Ausführungsbeispiele

Nachfolgend soll die Erfindung anhand einiger Ausführungsbeispiele eingehender erläutert werden. Die Ausführungsbeispiele sollen dabei die Erfindung beschreiben ohne diese zu beschränken.

Anhand von Zeichnungen wird die Erfindung näher erläutert. Dabei zeigen
- **Fig. 1**: Bauchelement des Systems zur Erfassung von Vitalfunktionen von Tieren,
- **Fig. 2**: Rückenelement des Systems zur Erfassung von Vitalfunktionen von Tieren,
- **Fig. 3**: Brustelement des Systems zur Erfassung von Vitalfunktionen von Tieren,
- **Fig. 4**: Schulterelement des Systems zur Erfassung von Vitalfunktionen von Tieren.

Die in den Figuren dargestellten oberen Teile der Bestandteile zeigen jeweils in Richtung Kopf des Hundes, die in den Figuren dargestellten unteren Teile der Bestandteile zeigen in Richtung Rute des Hundes. Die jeweils von oben nach unten eingezeichneten Längsachsen der Bestandteile verlaufen entlang der Wirbelsäule des Hundes und beziehen sich auf dessen Laufrichtung.

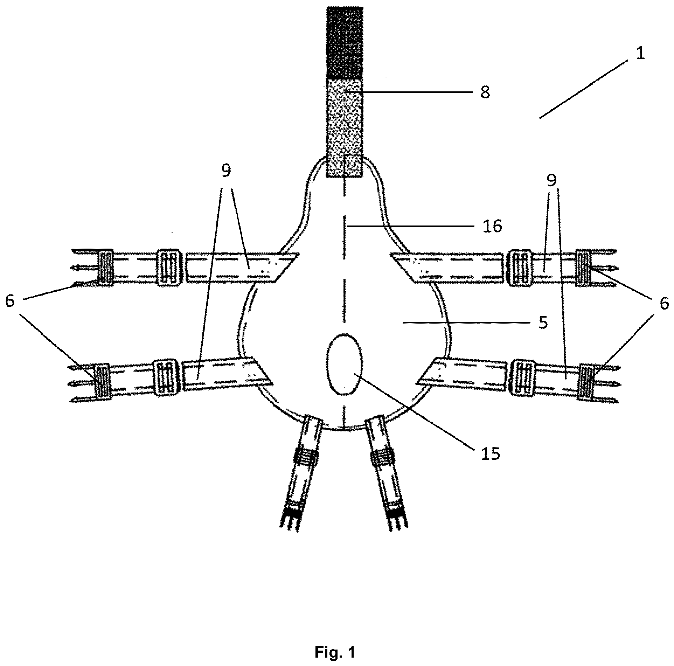

**Figur 1** zeigt ein Bauchelement 1, welches eine birnenförmige Auflagefläche 5 aufweist.

Das Bauchelement 1 weist vier punktuell daran angeordnete Gurtbänder 9 auf, welche spiegelsymmetrisch zu der Längsachse 16 des Bauchelementes 1 und voneinander beabstandet angeordnet sind. Dabei sind zwei Gurtbänder 9 auf der linken Seite und zwei Gurtbänder 9 auf der rechten Seite der Auflagefläche 5 des Bauchelementes 1 relativ zur Längsachse 16 angeordnet. Zusätzlich zu den vier punktuell angeordneten Gurtbändern 9 sind zwei schmaler ausgeführte Gurtbänder 9 an der in der Figur unteren Seite des Bauchelementes 1 dargestellt, durch welche die Rute des Hundes geführt wird. Die Gurtbänder 9 sind alle in der Länge durch Verstellschieber verstellbar.

In die Nähe der am Bauchelement 1 angeordneten Gurtbänder 9 sind jeweils Löcher (in der Figur nicht gezeigt) in der Auflagefläche 5 angebracht, welche einen Durchmesser von ca. 1 cm aufweisen. Diese Löcher sind für die Durchführung von Sensoren geeignet.

Zentral und symmetrisch um die Längsachse 16 im Bauchelement 1 angeordnet ist die Aussparung für die Durchführung des Geschlechtsorgans eines Rüden 15 vorgesehen.

Die Gurtbänder 9 sind mit dem Bauchelement vernäht. An jedem Gurtband 9 ist eine Klickschnalle 6 angeordnet, über welche das Bauchelement 1 mit dem Rückenelement 2 verbunden wird (Verbindung und Klickschnallen in der Figur nicht gezeigt).

Das Bauchelement 1 weist oben einen parallel zur Längsachse 16 verlaufenden Verbindungsgurt 8 als lösbares Verbindungsmittel auf, durch welche das Bauchelement 1 mit einem Brustelement 3 verbunden wird (Brustelement und Verbindung in der Figur nicht gezeigt). Der Verbindungsgurt 8 ist, ebenfalls wie die Gurtbänder 9, mit dem Bauchelement 1 vernäht.

Das Bauchelement 1 ist gepolstert und weist ein Abstandsgewirke auf (in der Figur nicht dargestellt).

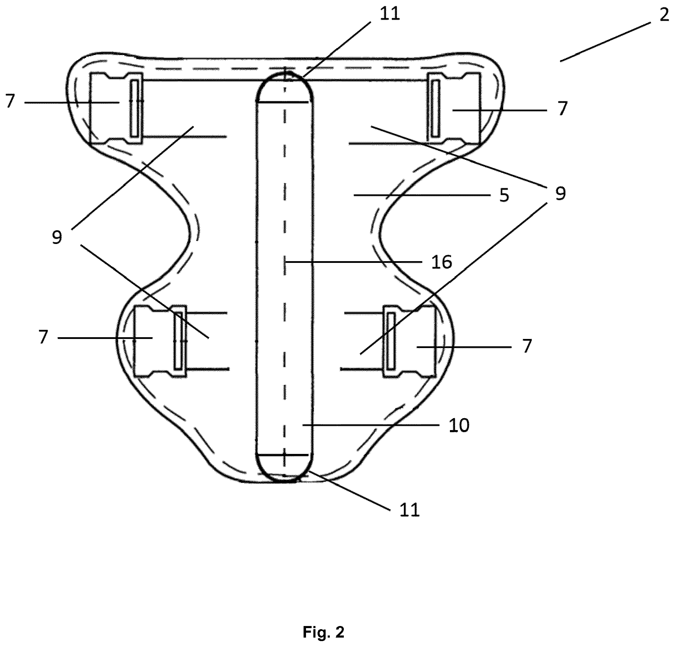

**Figur 2** zeigt ein Rückenelement 2, welches eine flächig ausgebildete Auflagefläche 5 aufweist. Das Rückenelement 2 weist vier Gurtbänder 9 auf, welche parallel senkrecht und voneinander beabstandet zur Längsachse 16 an das Rückenelement 2 angenäht sind und spiegelsymmetrisch zu der Längsachse 16 angeordnet sind. An jedem Gurtband 9 ist jeweils ein Gegenstück 7 zu der am Bauchelement 1 angebrachten Klickschnalle 6 angeordnet, über welche das Bauchelement 1 mit dem Rückenelement 2 lösbar verbunden wird (Verbindung in der Figur nicht gezeigt).

Am Rückenelement 2 ist eine Rückenschiene 10 aus Kunststoff befestigt, welche unter den über die Rückenschiene 10 des Rückenelementes 2 verlaufenden Gurtbändern 9 vernäht ist. Die Rückenschiene 10 verläuft durchgehend entlang der Längsachse 16 des Rückenelementes 2 und somit entlang der Wirbelsäule bis zum Schwanzansatz des Hundes.

Weiterhin weist das Rückenelement 2 an seinem oberen und unteren Ende entlang der Längsachse 16 jeweils einen D-Ring 11 auf. Dabei sind die D-Ringe 11 an zwei voneinander entlang der Längsachse 16 meist beabstandetsten Stellen des Rückelementes 2 mit einem Gurtband 9 vernäht. In die D-Ringe 11 kann jeweils das Ende eines Tragegriffs eingehakt werden (in der Figur nicht gezeigt).

Das Rückenelement 2 ist gepolstert (in der Figur nicht gezeigt).

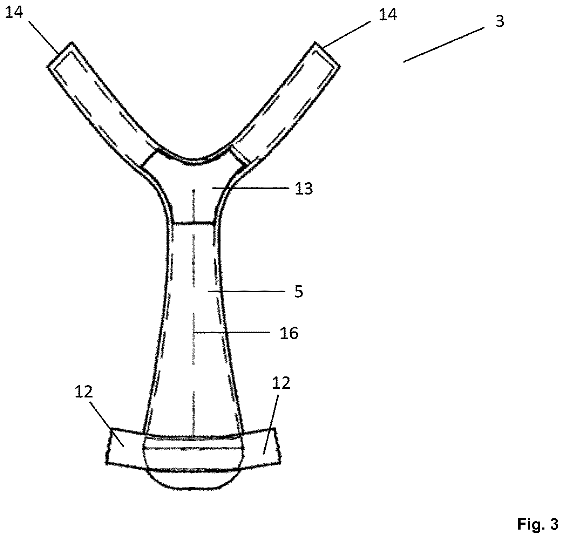

**Figur 3** zeigt ein Brustelement 3, welches eine stimmgabelförmige Auflagefläche 5 aufweist.

Das Brustelement 3 weist unten zwei punktuell am Brustelement 3 angeordnete Gurtbänder 12 auf, welche jeweils spiegelsymmetrisch zu der Längsachse 16 des Brustelementes 3 angeordnet sind. Dabei verlaufen die Gurtbänder 12 durchgehend über das Brustelement 3. An den Gurtbändern 12 ist jeweils eine daran angeordnete Klickschnalle angebracht (in der Figur nicht gezeigt).

Das Brustelement 3 weist an den Enden der beiden Zinken der stimmgabelförmigen Auflagefläche 5 jeweils eine lösbare Verbindung 14 auf, durch welche das Brustelement 3 mit dem Schulterelement 4 lösbar verbunden wird (Schulterelement in der Figur nicht gezeigt).

Das Brustelement 3 weist unten einen Klettverschluss für den vom Bauchelement 1 kommenden Verbindungsgurt 8 auf, durch welche das Brustelement 3 mit einem Bauchelement 1 verbunden wird (Verbindungsgurt und Klettverschluss in der Figur nicht gezeigt). Der Verbindungsgurt 8 ist dabei ebenfalls wie die Gurtbänder (9 und 12) mit dem Bauchelement 1 vernäht.

Das Brustelement 3 weist eine Brustbeinpolsterung 13 und ein Abstandsgewirke auf (Abstandsgewirke in der Figur nicht gezeigt).

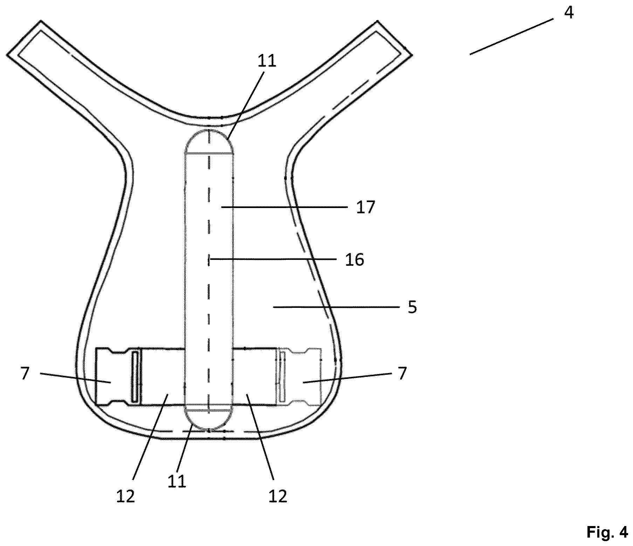

**Figur 4** zeigt ein Schulterelement 4, welches eine stimmgabelförmige Auflagefläche 5 aufweist, die breiter als beim Brustelement 3 ausgestaltet ist.

Das Schulterelement 4 weist unten zwei punktuell am Schulterelement 4 angeordnete Gurtbänder 12 auf, welche jeweils spiegelsymmetrisch zu der Längsachse 16 des Schulterelementes 4 angeordnet sind. Dabei verlaufen die Gurtbänder 12 durchgehend über das Schulterelement 4. An jedem Gurtband 12 ist jeweils ein Gegenstück 7 zu der am Brustelement 3 angebrachten Klickschnalle 6 angeordnet, über welche das Brustelement 3 mit dem Schulterelement 4 lösbar verbunden wird (Brustelement und Verbindung in der Figur nicht gezeigt).

Das Schulterelement weist weiterhin an den Enden seiner beiden Zinken einen Klickverschluss auf, durch welche das Schulterelement mit dem Brustelement lösbar verbunden wird (in der Figur nicht gezeigt).

Am Schulterelement 4 ist eine Schulterschiene 17 aus Kunststoff befestigt, welche unter den über die Schulterschiene 17 des Schulterelementes 4 verlaufenden Gurtbändern 12 vernäht ist. Die Schulterschiene 17 verläuft durchgehend entlang der Längsachse 16 des Schulterelementes 4 und somit entlang der Wirbelsäule des Hundes.

Weiterhin weist das Schulterelement 4 an seinem oberen und unteren Ende entlang der Längsachse 16 jeweils einen D-Ring 11 auf. Dabei sind die D-Ringe 11 an zwei voneinander entlang der Längsachse 16 meist beabstandetsten Stellen des Schulterelementes 4 mit einem Gurtband 12 vernäht. In die D-Ringe 11 kann jeweils das Ende eines Tragegriffs eingehakt werden (in der Figur nicht gezeigt). Die beiden Enden eines Tragegriffes können zudem auch in einen D-Ring 11 am Schulterelement 4 und in einen D-Ring 11 am Rückenelement 2 eingehakt werden, wodurch der Hund sowohl mittels des Schulterelementes 4 und des Rückenelementes 2 getragen werden kann.

Das Schulterelement 4 ist gepolstert (in der Figur nicht gezeigt).

### Bezugszeichen

- 1: Bauchelement
- 2: Rückenelement
- 3: Brustelement
- 4: Schulterelement
- 5: Auflagefläche
- 6: Klickschnalle
- 7: Gegenstück zur Klickschnalle
- 8: Lösbarer Verbindungsgurt (am Bauchelement) zum Brustelement
- 9: Lösbares Gurtband (am Bauchelement) zum Rückenelement
- 10: Rückenschiene
- 11: D-Ringe zur Befestigung von Tragegriffen
- 12: Lösbares Gurtband (am Brustelement) zum Schulterelement
- 13: Brustbeinpolsterung
- 14: Lösbare Verbindung (am Brustelement) zum Schulterelement
- 15: Aussparung für die Geschlechtsorgane von Rüden
- 16: Längsachse
- 17: Schulterschiene

## Patentansprüche

1. System zur Bestimmung des Vitalstatus von Tieren, umfassend:
zumindest eine erste Vorrichtung zur Erfassung von Vitalfunktionen, und
zumindest ein Mittel zur Signalübertragung,
wobei die erste Vorrichtung mindestens zwei voneinander beabstandete erste Sensoren aufweist.

2. System nach Anspruch 1, **dadurch gekennzeichnet, dass** die mindestens zwei voneinander beabstandeten ersten Sensoren als Pulsoxysensor und/oder Körpertemperatursensor und/oder Temperatursensor und/oder Schrittzahlsensor und/oder bioelektrischer Widerstandssensor ausgebildet sind.

3. System nach einem der Ansprüche 1 bis 2, **dadurch gekennzeichnet, dass** die erste Vorrichtung weiterhin zumindest zwei voneinander beabstandete zweite Sensoren aufweist, die von den mindestens zwei voneinander beabstandeten ersten Sensoren verschieden sind, wobei die ersten Sensoren und die zweiten Sensoren ausgewählt sind aus einem Pulsoxysensor und/oder Körpertemperatursensor und/oder Temperatursensor und/oder Schrittzahlsensor und/oder bioelektrischer Widerstandssensor.

4. System nach einem der Ansprüche 1 bis 3, **dadurch gekennzeichnet, dass** es weiterhin eine zweite Vorrichtung zur Positions- und Standortbestimmung von Tieren umfasst.

5. System nach einem der Ansprüche 1 bis 4, **dadurch gekennzeichnet, dass** es weiterhin eine dritte Vorrichtung umfasst, ausgewählt aus Gassensoren und/oder einem Modul zur eindeutigen Besitzeridentifikation und/oder einem Modul zur Deaktivierung und Sperrung.

6. System nach einem der Ansprüche 1 bis 5, weiterhin umfassend eine Positionierungseinrichtung, an welcher zumindest die mindestens zwei voneinander beabstandeten ersten und/oder zweiten Sensoren und/oder das zumindest eine Mittel zur Signalübertragung angeordnet sind.

7. System nach einem der Ansprüche 1 bis 6, **dadurch gekennzeichnet, dass** die mindestens zwei voneinander beabstandeten ersten und/oder zweiten Sensoren punktuell oder flächendeckend in der Positionierungseinrichtung eingebracht sind.

8. System nach einem der Ansprüche 1 bis 7, **dadurch gekennzeichnet, dass** die mindestens zwei voneinander beabstandeten ersten und/oder zweiten Sensoren entlang der Herzachsenlagen des Tieres angebracht sind.

9. System nach einem der Ansprüche 1 bis 8, **dadurch gekennzeichnet, dass** die Bestandteile des Systems zur Erfassung von Vitalfunktionen von Tieren mittels Verbindungsstücke mit der Positionierungseinrichtung lösbar verbindbar ausgebildet sind.

10. System nach einem der Ansprüche 1 bis 9, **dadurch gekennzeichnet, dass** die Positionierungseinrichtung als Stabilisierungsvorrichtung zur medizinischen Behandlung, zur Entlastung, Stabilisation, Sicherung sowie dem waagerechten Anheben und Transport von Tieren ausgestaltet ist, umfassend mindestens ein Bauchelement und ein Rückenelement, welche durch Verbindungselemente lösbar verbindbar ausgestaltet sind.

11. Verfahren zur Bestimmung des Vitalstatus von Tieren, wobei die Vitalfunktionen durch eine erste Vorrichtung mit mindestens zwei voneinander beabstandeten ersten Sensoren erfasst werden, wobei die erfassten Daten durch zumindest ein Mittel zur Signalübertragung an eine Datenverarbeitungseinrichtung übermittelt, verarbeitet und ausgewertet werden und von der Datenverarbeitungseinrichtung an ein externes Ausgabegerät übermittelt werden, welche den Vitalstatus von Tieren ausgibt.

12. Verfahren nach Anspruch 11, **dadurch gekennzeichnet, dass** die Daten der Vitalfunktionen durch mindestens zwei voneinander beabstandete erste Sensoren und/oder zwei voneinander beabstandete zweite Sensoren erfasst werden.

13. Verfahren nach Anspruch 11 oder 12, **dadurch gekennzeichnet, dass** Daten zu den Vitalfunktionen durch eine zweite Vorrichtung und/oder dritte Vorrichtung erfasst werden.

14. Verwendung eines Systems zur Erfassung des Vitalstatus von Tieren nach einem der Ansprüche 1 bis 10 sowie Verwendung eines Verfahrens zur Bestimmung des Vitalstatus von Tieren nach einem der Ansprüche 11 bis 13.
